# EUROPEAN PATENT APPLICATION

(11) **EP 2 080 535 A1**
(43) Date of publication of application: **22.07.2009**
(21) Application number: 07831203.0
(22) Date of filing: 05.11.2007
(51) Int. Cl.: A61M 25/00, A61L 29/00

(54) **CATHETER TUBE FOR MEDICAL USE**

(30) Priority: 07.11.2006 JP 2006301599
(71) Applicant: Kaneka Corporation, Kita-ku Osaka-shi, Osaka 530-8288 (JP)
(72) Inventor: TSUKUMO, Kazutaka, Settsu-shi Osaka 566-0072 (JP); MURATA, Takahiro, Settsu-shi Osaka 566-0072 (JP)
(74) Representative: Gille Hrabal Struck Neidlein Prop Roos
(86) International application number: PCT/JP2007/071470
(87) International publication number: WO 2008/056625

(57) **Abstract**

A catheter tube for medical use which comprises an inner resin layer, a reinforcing material layer located on the inner resin layer and an outer resin layer located on the reinforcing material layer, **characterized in that** the reinforcing material layer (3) is composed of twisted yarns (7). Thus, it becomes possible to provide a catheter tube for medical use that is excellent in the shape recovery (memory) properties after the formation of a kink or a puncture and the flexibility at the front edge.

## Description

### TECHNICAL FIELD

The present invention relates to a medical catheter tube, more specifically, to a medical catheter tube for example for injection of a selective contrast medium or a thrombolic substance or for thrombotic suction or peripheral angiogenesis.

### BACKGROUND ART

Catheter tubes are hollow medical devices that are used as inserted in the cavity, duct, blood vessel, and others in the body, specifically for selective injection of contrast medium, thrombolic substance or others, thrombotic suction, recirculation of clogged vessel, vasodilatation, and the like. There are a variety of requirements on these catheter tubes, but such a catheter mainly demands superior usability allowing rapid, accurate and selective insertion thereof, for example, into thin complicated-patterned blood vessel.

For example, the catheter tube also demands favorable kink resistance prohibiting folding or deforming in the curved or complexly bent area of blood-vessel when the distal end of catheter tube reaches a desired position and then the guide wire is removed, and also favorable distal-region flexibility keeping the shape thereof favorable for the blood vessel without damaging it. Because blood vessels are bent complexly, also important are the torque-transmitting efficiency of conveying force from the proximal end region to the distal end region smoothly during revolving insertion and also the shape recovery efficiency of recovering the lumen original shape even if the catheter is folded or deformed.

To satisfy the requirements above, traditional catheters generally have a three-layer structure consisting of a flexible internal layer, a reinforcing layer formed on the internal layer and a flexible external layer additionally formed on the reinforcing layer, wherein the catheter is relatively rigid in the proximal end region and becomes softer gradually in the area closer to the distal end region.

Patent Document 1 discloses a catheter having shape-memory property at body temperature at least in the catheter distal end region that regains its original shape at body temperature after deformation of the catheter distal end region. In the disclosed catheter structure containing a metal tube connected to the resin tube in the distal end region, there may be kink generation in the connection region because of large difference in hardness there, and thus, the catheter does not have a structure sufficiently resistant to kink. In addition, no twisted yarn is considered as the reinforcing material.

Patent Document 2 discloses a dilation catheter, comprising a relatively rigid tubular proximal shaft, a tubular distal shaft less rigid than the proximal shaft, a tubular intermediate region formed between the proximal and distal shafts, a hub connected to a site close to the proximal end of the proximal shaft for connection with a pressure application device, a balloon formed fluidally communicative to the distal end region of the distal shaft to which the pressure from the hub is applied, and a lumen for insertion of a guide wire having a distal opening to the distal side of the balloon distal end and a proximal opening to the proximal side of the balloon proximal end, wherein a reinforcing material of braid of linear materials is embedded in the intermediate region.

In the catheter having a configuration containing an embedded reinforcing material of braid, there are regions where the inclination angle of the braid is relatively lower and regions where it is larger. Although it is possible to prevent kink effectively in the regions when the inclination angel of the linear material is small, the tube is kinked or deformed, making the guide wire stuck or prohibiting injection of contrast medium or thrombolic substance in the regions where the inclination angel of the linear material is large, when the catheter tube is inserted into the complexly bent region in blood vessel and pushed or pulled under excessive force, during operation. In addition, use of shape recovery property or twisted yarn is not considered.

Patent Document 3 discloses prior art concerning shape recovery of medical catheter tube. However, the kink resistance or the shape recovery is provided by a circular element, and use of a twisted yarn as reinforcing layer material, a characteristic feature of the present invention, is not considered.

Patent Document 4 discloses prior art concerning reinforced catheter tubes. However, although use of a core-containing multifilament as the reinforcing material is disclosed there, use of twisted yarn is not considered and there is no description of the effects and embodiments.

Patent Document 5 discloses a catheter in which resin wires are wound helically and the shaft stiffness is controlled by modification of the winding pitch, but the reinforcing material used is a ribbon-shaped Kevlar and shape recovery or use of twisted yarn is not considered.

Similarly, Patent Document 6 describes a catheter device having a structure in which braid or coil is formed by using a monofilament or a multifilament yarn, but shape recovery or use of twisted yarn is not considered.
Patent Document 1: JP-A No. 2001-058010
Patent Document 2: JP-A No. 2001-095923
Patent Document 3: US 2005/0165366
Patent Document 4: WO 1993/023105
Patent Document 5: US 4516972
Patent Document 6: US 2004-0138644

### Disclosure of the Invention

### Problems to be Solved by the invention

An object of the present invention is to provide a medical catheter tube superior in shape recovery (memory) efficiency and bending flexibility.

### Means to Solve the Problems

Accordingly, the present invention is related to:
(1) A medical catheter tube, comprising an internal resin layer, a reinforcing material layer formed on the internal resin layer and an external resin layer formed on the reinforcing material layer, wherein the reinforcing material layer comprises twisted yarns;

(2) The medical catheter tube described above, wherein single yarns forming the twisted yarns comprise a nonmetal wire;

(3) The medical catheter tube described above, wherein the nonmetal wire is a glass fiber;

(4) The medical catheter tube described above, wherein the shape recovery (memory) rate in the folded region after folding 180° is 90% or more;

(5) The medical catheter tube described above, wherein the nonmetal wire contains a radiopaque element;

(6) The medical catheter tube described above, wherein the radiopaque element is one or more elements selected from barium, tantalum, tungsten, iridium, platinum, gold, and bismuth;

(7) The medical catheter tube described above, wherein the single yarns for the twisted yarn comprise a metal wire;

(8) The medical catheter tube described above, wherein the single yarns for the twisted yarn comprise a metal wire and a nonmetal wire;

(9) The medical catheter tube described above, wherein the nonmetal wire has a tensile strength of 13.2 cN/dtex or more and a structure having a melting liquid crystal polymer as core and a flexible polymer as sheath; and

(10) The medical catheter tube described above, wherein the shape recovery (memory) rate in the folded region after folding 180° is 75% or more.

### Advantageous Effects of the Invention

The present invention, which comprises twisted yarns in the reinforcing material layer, provides a medical catheter tube superior in shape recovery (memory) efficiency after kink or deformation and also in the bending flexibility in the distal end region.

### BRIEF DESCRIPTION OF THE DRAWINGS

Figure 1 is a schematic explanatory drawing illustrating an embodiment of the production method for a medical catheter according to the present invention having four kinds of resin tubes different in Shore D hardness for the external resin layer placed closely in contact with each other.
Figure 2 is a schematic explanatory drawing illustrating the catheter in the state having a heat-shrinkable tube formed thereon.
Figure 3 is a schematic view illustrating the catheter in the state having a radiopaque marker placed thereon after removal of the heat-shrinkable tube.
Figure 4 is a schematic view illustrating the medical catheter according to the present invention.
Figure 5 is a schematic view illustrating part of a reinforcing material layer, i.e., a tow of three twisted yarns respectively consisting of three single yarns, and a photograph of such a reinforcing layer braid formed.

### EXPLANATION OF NUMERALS

1 Metal core wire
2 Internal resin layer
3 Reinforcing material layer
4 External resin layer
4a Highest-Shore D hardness external layer tube
4b High-Shore D hardness external layer tube
4c Low-Shore D hardness external layer tube
4d Lowest-Shore D hardness external layer tube
5 Heat-shrinkable tube
6 Marker
7 Twisted yarn

### BEST MODE OF CARRYING OUT THE INVENTION

Hereinafter, the medical catheter tube according to the present invention will be described, but it should be understood that the present invention can be modified in various ways within the scope of the present invention specified in its claims.

### 1. Medical catheter tube

The medical catheter tube according to the present invention is a medical catheter tube having an internal resin layer, a reinforcing material layer formed on the internal resin layer, and an external resin layer formed on the reinforcing material layer, **characterized in that** the reinforcing material layer comprises twisted yarns. Advantageously in the structure, the medical catheter tube is superior in shape recovery (memory) efficiency after kink or deformation and also in bending flexibility. A metal wire or a nonmetal wire (e.g., glass fiber or resin fiber) may be used as a single yarn. The twisted yarn means not only a twist of multiple single yarns but also a twist or combination of a plurality of the twist of multiple single yarns (doubly twisted yarns). The twisted yarn can be formed in combination of two or more wires selected from metal and nonmetal wires.

If a nonmetal wire such as glass fiber or resin wire is used, it is possible to make the medical catheter tube radiopaque by adding an X-ray-blocking element to the glass composition as particles.

### 2. Internal resin layer

The internal resin layer constituting the medical catheter tube according to the present invention preferably has a tubular structure, and the shape and size of the tubular cross section are preferably those permitting insertion of the guide wire.

Examples of the materials for the internal resin layer include fluorine resins such as polytetrafluoroethylene, tetrafluoroethylene-perfluoroalkyl vinyl ether copolymers, tetrafluoroethylene-hexafluoropropylene copolymers and ethylene-tetrafluoroethylene copolymers. Among them, materials favorably lubricious to the guide wire passing through the internal resin layer lumen in final product include polytetrafluoroethylene, tetrafluoroethylene-perfluoroalkyl vinyl ether copolymers and the like. If polytetrafluoroethylene is used, the catheter is usually baked, after the additives are previously treated for example by drying. A resin more lubricious such as polyethylene may be used in molding, for improvement in processability.

The internal resin layer coated on the reinforcing material layer is preferably adhesive enough to the reinforcing material layer. In addition, the internal resin layer, which becomes in contact locally with the external resin layer formed thereon, is also adhesive enough to the external resin layer. Thus for improvement in adhesiveness, the surface of the internal resin layer may be roughened or modified by one or more methods selected from mechanical methods (surface roughening for example with sand paper) chemical methods (use of defluorinating agent such as sodium naphthalene + dimethylether) and electrical methods such as plasma processing and the like.

### 3. Reinforcing material layer

The medical catheter tube according to the present invention has a reinforcing material layer of twisted yarns formed on the internal resin layer. The reinforcing material layer is preferably formed in a braid structure.

The braid structure of the reinforcing material layer of catheter has roles of providing pressure resistance, torque-transmitting efficiency, and additionally kink resistance.

There are many possible shape of the braid structure, such as 1 over 1 under structure involving crossing of every single wire, 2 over 2 under structure involving crossing of every two wires, structure formed with a tow of multiple twisted yarns, structure formed with 8 to 32 tows, and the combination thereof. A braid in the 1 over 1 under structure, wherein the number of metal wires per tow is 2 to 6, and 16 to 24 tows are used, is most preferable, from the viewpoints of insertion efficiency, pressure resistance, and kink resistance.

A metal or nonmetal wire is used favorably as the wire for the reinforcing material layer, because it is superior in dimensional stability and tensile strength.

### 4. Radiopaque substance

If a glass or resin fiber is used as the nonmetal wire in the medical catheter tube according to the present invention, a glass or resin fiber containing a radiopaque element dispersed therein is preferably used, to make the medical catheter tube more visible.

The radiopaque element is not particularly limited, but preferably one or more elements selected from barium, tantalum, tungsten, iridium, platinum, gold and bismuth.

### 5. Single yarn

The single yarn for use may be a metal wire, a nonmetal wire (e.g., glass fiber, resin wire); if a glass fiber is used as the single yarn, the single yarn preferably has a diameter (as of circular cross section) of 0.001 mm to 0.010 mm, preferably 0.002 mm to 0.005 mm, and a twisted yarn desirably has 5 to 200 said glass fiber single yarns, preferably 10 to 100 single yarns, for reduction of the thickness of the catheter tube while preserving its lumen diameter.

Examples of the materials for the metal wire include stainless steel, tungsten, copper, nickel, titanium, piano wire, cobalt-chromium-based alloy, nickel-titanium-based alloy (superelastic alloy), copper-zinc-based alloy, amorphous alloy and the like. The material is preferably less visible than the radiopaque marker, to make the radiopaque marker formed later more visible, and thus, for that reason and also for improvement in processability and safety, use of stainless steel, tungsten, a nickel-titanium alloy (superelastic alloy), or a cobalt-chromium alloy is preferable.

The material for the nonmetal wire is not particularly limited, and examples thereof include the wire having the core of a melting liquid crystal polymer and the sheath of a melting liquid crystal polymer (islands) and a flexible polymer (sea), as disclosed for example in JP-A No. 2005-34513, and additionally, polyesters such as polyethylene terephthalate, polybutylene terephthalate and polymethylene terephthalate; polyolefins such as polyethylene and polypropylene; rigid polyvinyl chloride, polyamide, polyimide, polystyrene, thermoplastic polyurethane, polycarbonate, ABS resins, acrylic resins, polymethyl methacrylate, polyacetal, polyarylate, polyoxymethylene, high-strength polyvinylalcohol, fluoroplastics, polyvinylidene fluoride, polytetrafluoroethylene, ethylene-vinyl acetate hydrolysates, polysulfone, polyether sulfone, polyether ketone, polyphenylene oxide, polyphenylene sulfide, aromatic polyaramides such as Kevlar, and the like, as well as the polymer alloys containing the polymers above.

As for the single-yarn size of the metal wire or the nonmetal wire (excluding glass wire), a twisted yarn containing 3 to 30, preferably 3 to 7 single yarns having a the diameter as of approximately circular cross section of 0.008 mm to 0.040 mm, preferably 0.010 mm to 0.030 mm that are twisted at a pitch of 3 to 60 times, preferably 5 to 30 times larger than the filament diameter is used, for reduction of the thickness of the catheter tube while preserving its lumen diameter. The direction of twisting is not particularly limited, and thus, may be rightward or leftward.

It is possible to improve the shape recovery (memory) efficiency after kink or deformation of the catheter tube, and stability of operation, by using a metal or nonmetal wire of a twisted yarn of multiple single yarns in the reinforcing material layer of the medical catheter tube according to the present invention.

The diameter of the single yarn can be determined, for example, by using a micrometer.

### 6. External resin layer

The medical catheter tube according to the present invention has an external resin layer additionally formed on the reinforcing material layer. The material for the external resin layer is not particularly limited, and examples thereof include various elastomers such as polyamide elastomers, polyester elastomers, polyurethane-elastomers, polystyrene elastomers, fluorine-based elastomers, silicone rubbers and latex rubbers, and two or more of them in combination may also be used favorably.

The polyamide elastomer is typically a block copolymer having an aliphatic or aromatic polyamide, such as nylon 6, nylon 64, nylon 66, nylon 610, nylon 612, nylon 46, nylon 9, nylon 11, nylon 12, N-alkoxymethyl-modified nylon, hexamethylenediamine-isophthalic acid condensation polymer, or meta xyloyldiamine-adipic acid condensation polymer, as the hard segment and a polymer such as polyester or polyether as the soft segment, but it is a concept additionally including polymer alloys thereof (polymer blend, graft polymerization, random polymerization, etc.) of the polyamide with a more flexible resin, modified polyamides that are softened for example with a plasticizer, and the mixture thereof.

The polyester elastomer is typically a block copolymer of saturated polyester such as polyethylene terephthalate or polybutylene terephthalate and a polyether or polyester, but is a concept additionally including alloys of these polymers, modified saturated polyester softened, for example, with a plasticizer, and the mixture thereof. The material favorably used from the viewpoints of processability and flexibility is a polyamide elastomer, and a typical example thereof is "PEBAX" (trade name) manufactured by ARKEMA.

If the resin is a polyether ester amide elastomer, such as PEBAX (registered trade name), the Shore D hardness of the polyether ester amide elastomer material is normally proportional with the weight rate of the hard segment in the polyether ester amide elastomer material. It is thus possible to use the weight rate of the hard segment in polyether ester amide elastomer as an indicator of Shore D hardness, by calculating it by the following method and comparing it with that of standard sample. The weight rate of the hard segment in polyether ester amide elastomer is obtained by measuring the weights of the polyamide, ester and polyether regions by H¹-NMR and calculating the ratio of polyamide region weight/(polyamide region weight + ester region weight + polyether region weight).

On the other hand, it is preferable to make the Shore D hardness of the medical catheter tube according to the present invention decrease stepwise or gradually in the direction from the proximal end region to the distal end region.

### 7. Production method

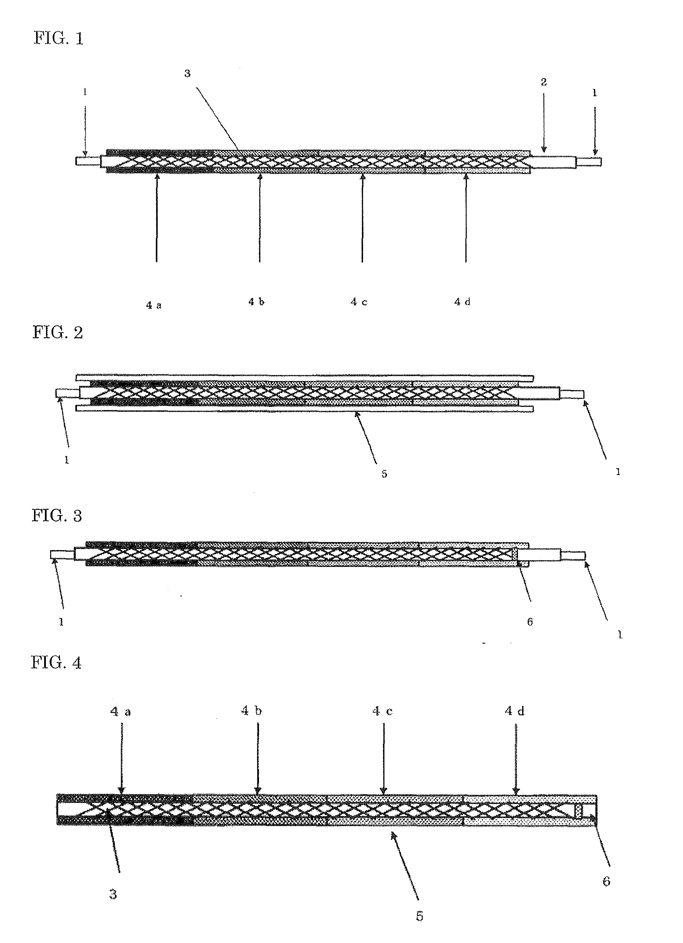

The method of producing the medical catheter tube according to the present invention is not particularly limited, but an example of the production method will be described below with reference to Figures 1 to 5.

First, a metal core wire 1 is made available. The metal core wire 1 may be supplied, for example, in the shape wound around a reel, and normally, the external diameter is almost identical with the lumen diameter of the catheter produced. The material for the metal core wire 1 is not particularly limited, but preferably a metal-plated copper wire or a stainless steel wire.

A resin composition for the internal layer is then extruded around the metal core wire 1, for example, by extruder, to form an internal resin layer 2. The condition of the extrusion coating is selected properly depending on the composition of the resin composition for internal layer and the kind of the metal core wire used.

The metal core wire 1 carrying the internal resin layer 2 formed is then placed, for example, in a braiding machine, and a reinforcing material layer 3 of twisted yarns, more preferably a reinforcing material layer 3 of twisted yarns in a braid structure, is formed on the internal resin layer 2. Any known machine may be used as the braiding machine.

An external resin layer 4 is then formed on the reinforcing material layer 3.

The method of forming the external resin layer 4 is not particularly limited, but preferably used is, for example, a method of forming an external resin layer 4 by extrusion coating for example by using an extruder on the reinforcing material layer 3, or a method of placing a tube for external resin layer 4 previously formed by extrusion molding on the reinforcing material layer 3 and then, tightly coating the tube for external resin layer 4 around it by shrinkage by heating and the like. For alternation of the Shore D hardness of the external resin layer stepwise or gradually in the direction from the proximal end region to distal end region, the latter method, if used, may be a method of preparing multiple external resin layer tubes different in hardness, placing an external resin layer tube higher in hardness in the proximal end region, placing softer external resin layer tubes toward the distal end region, covering these external resin layer tubes with a heat-shrinkable tube 5, and integrating the internal resin layer, the reinforcing material layer, and the external resin layer, or the former method, if used, may be a method of forming external resin layers softer stepwise in the direction from the proximal end region to the distal end region by placing a reel of the reinforcing material layer in an extruder having a valve mechanism and feeding and discharging resins different in hardness into and out of the extrusion channel sequentially by continuous extrusion.

Figure 1 shows the configuration of a catheter that are formed by the former method, having four kinds of external resin layer tubes 4a to d different in Shore D hardness that are placed as they are tightly connected to each other, as the external resin layer (in common medical catheter tubes, the Shore D hardness preferably decreases from the proximal end to the distal end and, in Figure 1 the Shore D hardness in the external resin layer tubes is preferably in the order of 4a>4b>4c>4d. However, the order may be altered as needed). The Shore D hardness of the external resin layer used is preferably in the range of 20 to 80, considering use in complicated blood vessel. The Shore D hardness, as used in the present description, is a value determined by using a durometer type D according to ISO 7619.

Subsequently as shown in Figure 2, the composite is then entirely covered with a heat-shrinkable tube 5 that shrinks in its diameter when heated.
The material for the heat-shrinkable tube 5 is preferably polytetrafluoroethylene, a perfluoroethylene-propene copolymer or the like.

The heat-shrinkable tube 5 covering the entire catheter is then removed and part of the reinforcing material layer 3 and the external layer tube 4d having the lowest Shore D hardness, which correspond to the marker region and the soft region of the catheter, is removed to expose the internal resin layer 2.

Hereinafter, the position of the marker 6 of a radiopaque metal will be described (see Figure 3). The position of the marker is a position 0.5 mm to 2.0 mm separated from the most distal end region of the catheter. The marker is for example a radiopaque metal tube, a metal single yarn, or the like. These parts may be placed after internal resin layer is exposed, as described above, or may be formed on the internal resin layer 2 previously.

When a metal tube is used for prevention of exposure of the marker from the external resin layer, the thickness thereof is preferably 0.005 mm to 0.060 mm, while when a metal single yarn is used, the diameter thereof is preferably 0.0055 mm to 0.060 mm.

The material for the radiopaque metal tube or the single yarn for use may be a tungsten-based metal, a platinum-based metal or a gold-based metal. The tungsten-based metals include pure tungsten and W-45Mo alloy, W-5Mo-5Ni(Co, Fe) alloy, W-Re-based alloys, W-ThO₂ alloys, and alloys thereof with tungsten, copper, carbon or the like. The platinum-based metals include platinum, alloys of platinum with tungsten, rhodium, iridium, osmium, palladium, ruthenium or the like. The gold-based metals include pure gold and alloy of gold with copper, silver, rhodium, iridium, osmium, palladium, ruthenium or the like.

Then, a soft external resin layer tube 4d is placed again on the radiopaque metal marker 6 and the internal-layer tube.

The soft external layer tube 4d thus formed again is covered on its periphery with a heat-shrinkable tube that shrinks in its diameter when heated.

Then, the catheter is heated to the shrinkage temperature of the heat-shrinkable tube by a heater or by irradiation with a high-frequency electromagnetic wave, forming the marker region and the soft region, as integrated with the internal-layer tube, the radiopaque metal marker 6, and the external resin layer tube 4d.

After removal of the heat-shrinkable tube, the catheter tube surface is preferably covered with a hydrophilic (or water-soluble) polymer substance. In this way, it is possible to reduce the friction coefficient of the catheter, making it more lubricant, to increase the lubricity of the catheter tube further even when the outermost surface of the catheter tube becomes in contact with blood, physiological saline, or the like, and consequently, to improve insertion efficiency, compatibility, kink resistance, and stability further. Examples of the hydrophilic polymer substances include the following natural or synthetic polymer substances and the derivatives thereof. Particularly favorable are cellulosic polymer substances (such as hydroxypropylcellulose), polyethylene oxide-based polymer substances (such as polyethylene glycol), maleic anhydride-based polymer substances (maleic anhydride copolymers such as methylvinylether-maleic anhydride copolymer), acrylamide-based polymer substances (such as polyacrylamide), and water-soluble nylons, because these materials give a low friction coefficient consistently.

Finally, the metal core wire (core metal) 1 is withdrawn; both terminals are cut, for example, with a diamond cutter revolving at high speed; and the proximal end region is finished to a single plane, to give a catheter tube (Figure 4).

It is possible to obtain a catheter tube favorable in gradation in stiffness and flexibility and thus, favorable to various access routes, by adjustment of the length of the external layer tubes different in Shore D.

In addition, it is possible to obtain a desired medical catheter by connecting a hub in a suitable shape to the proximal end region. The medical catheters include, for example, guiding catheter, micro catheter, balloon catheter, thrombus suction catheter, and the like.

The medical catheter tube may be used as it is, as described above, or, if appropriate, part of the medical catheter tube may be heated previously by heater or steam to form a shape such as curved region.

### EXAMPLES

Hereinafter, the present invention will be described in detail with reference to Examples and Comparative Examples, but it should be understood that the present invention is not restricted thereby.

### (Example 1: Braid structure formed with twisted yarns of nonmetal wire)

A polytetrafluoroethylene (herein after, PTFE) having a thickness of 0.030 mm is formed around a metal-plated copper wire (external diameter ϕ: 0.52 mm) by coating extrusion. The PTFE-coated metal-plated copper wire is then placed in a braiding machine, and a tow of glass fiber having 100 single yarns of a diameter ϕ of 0.004 mm that is twisted at a pitch of 25 mm is formed, and a braid tube in a braid structure of 16 tows is formed (i.e., the braid structure corresponds to a reinforcing material layer formed by 16 twisted yarns consisting of 100 twisted single yarns). Then, the braid tube of 1600 mm in length is cut off. The pitch on the braiding machine is set to 1.0 mm. The braid tube is then covered with a tube having a Shore D hardness 25 of a polyamide elastomer (Pebax (registered trade name) manufactured by ARKEMA, the same shall apply hereinafter) previously formed by extrusion molding and additionally with a fluorine-based heat-shrinkable tube thereon. The catheter is heated to the melting point of the polyamide elastomer or higher, allowing thermal fusion of the external layer by the heat shrinkage force of the heat-shrinkable tube, and then cooled, and the heat-shrinkable tube is removed. Finally, withdrawal of the metal-plated copper wire as a core wire gives a medical catheter tube.

### (Example 2: Braid structure formed with twisted yarns of metal wire)

A tube is formed in a similar manner to Example 1, except that a metal wire of SUS304 is used as the braid wire, and the braid tube in a braid structure of 16 tows is formed, each tow comprising three metal wires without being twisted, each metal wire comprising three single yarns having diameter ϕ of 0.011 mm twisted at a pitch of 0.7 mm.

### (Comparative Example 1: Braid structure formed with nonmetal wires)

A tube is formed in a similar manner to Example 1, except that a nonmetal wire of glass fiber is used as the braid wire and a braid tube in the 16-braid structure, each braid being a glass fiber tow of 100 single yarns having a diameter ϕ of 0.004 mm.

### (Comparative Example 2: Braid structure formed with metal wires)

A tube is formed in a similar manner to Example 1, except that a metal wire of SUS304 is used as the braid wire, and the braid tube is formed in a 16-braid structure, each braid containing three metal wire tows, each tow containing three metal wires having a single yarn diameter ϕ of 0.011 mm.

### (Example 3: Braid structure formed with twisted yarns of metal wire)

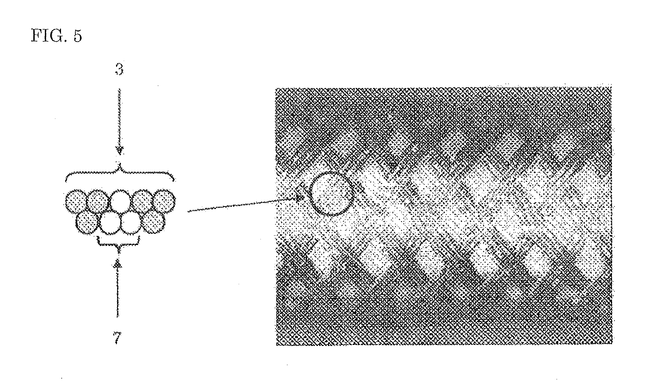

A polytetrafluoroethylene (PTFE) having a thickness of 0.020 mm is formed on a metal-plated copper wire (external diameter ϕ: 1.10 mm) by extrusion coating. The PTFE-coated metal-plated copper wire is then placed in a braiding machine; a tow of three metal wires having a single yarn diameter ϕ of 0.020 mm twisted at a pitch of 0.7 mm is formed; and a braid tube in a 16-braid structure is formed (see Figure 5). Then, the braid tube of 1600 mm in length is cut off. The pitch on the braiding machine is set to 2.5 mm. The braid tube is then covered with a tube having a Shore D hardness 35 of a polyamide elastomer (Pebax (registered trade name) manufactured by ARKEMA, the same shall apply hereinafter) previously formed by extrusion molding and additionally with a fluorine-based heat-shrinkable tube thereon. The catheter is heated to the melting point of the polyamide elastomer or higher, allowing thermal fusion of the external layer by the heat shrinkage force of the heat-shrinkable tube, and then cooled, and the heat-shrinkable tube is removed. Finally, withdrawal of the core wire gives a medical catheter tube.

### (Comparative Example 3: Braid structure formed with metal wires)

A tube is formed in a similar manner to Example 3, except that the metal wire of SUS304 is used as the braided wire and a braid tube in a 16-braid structure is formed with a tow of three metal wires having a single yarn diameter ϕ of 0.020 mm.

### (Comparative Example 4: Braid structure formed with metal wires)

A tube is formed in a similar manner to Example 3, except that the metal wire of SUS304 is used as the braided wire and a braid tube in a 16-braid structure is formed with a tow of metal wire having a width of 0.100 mm and a thickness of 0.030 mm.

Measurement of shape recovery (memory) rate, bending test, and the internal and external diameter were carried out by using each of the catheter tubes formed in Examples 1 to 3 and Comparative Examples 1 to 4.

### <Method of measuring shape recovery (memory) rate>

The width (external diameter) of the catheter tube formed is determined by using the function of determining dimension of Micro Highscope (manufactured by KEYENCE). The catheter tube is then folded 180° and then straightened to the original state, and the internal diameter of the tube in the most damaged kink region is determined by using the function of determining dimension of Micro Highscope. The change in width of the tube between before and after folding is used as the shape recovery (memory) rate. The measured catheter tube number n is 5.

### <Bending test method>

The load applied when a catheter tube is inserted to a depth of 1.0 mm by using a load cell 1N at an inter-support distance of 15 mm in E-Z Test (manufactured by Shimadzu Corp.) is used as the physical property value. The catheter tube is softer and favorable, when the value is smaller. The measured catheter tube number n is 3.

### <Method of measuring internal diameter>

Pin gauges are inserted into a catheter tube in the increasing order in diameter, and the diameter of the pin gauge before that not entering the catheter tube is used as the observed value.

### <Method of measuring external diameter>

The average of five external average diameters, as determined biaxially by using a laser external diameter meter (manufactured by KEYENCE), is used as the observed value.

Table 1 summarizes the shape recovery (memory) rate and bending test evaluation results of the catheter tubes having an internal diameter ϕ of 0.51 mm ±0.02 mm and an external diameter ϕ of 0.70 mm ±0.02 mm, while Table 2 summarizes the shape recovery (memory) rate and the bending test evaluation results of the catheter tubes having an internal diameter ϕ of 1.09 mm ±0.02 mm and an external diameter ϕ of 1.35 mm ±0.02 mm.

**[Table 1]**

| | External layer 25D Shape recovery (memory) rate (%) | External layer 25D Bending load (N) | Internal diameter (mm) | External diameter (mm) |
|---|---|---|---|---|
| Example 1 | 91.7 | 0.025 | 0.51 | 0.72 |
| Example 2 | 78.3 | 0.021 | 0.51 | 0.69 |
| Comparative Example 1 | 80.5 | 0.032 | 0.51 | 0.71 |
| Comparative Example 2 | 63.5 | 0.020 | 0.51 | 0.70 |

**[Table 2]**

| | External layer 35D Shape recovery (memory) rate (%) | External layer 35D Bending load (N) | Internal diameter (mm) | External diameter (mm) |
|---|---|---|---|---|
| Example 3 | 77.5 | 0.15 | 1.09 | 1.34 |
| Comparative Example 3 | 61.8 | 0.17 | 1.09 | 1.33 |
| Comparative Example 4 | 71.7 | 0.21 | 1.09 | 1.35 |

As shown in Tables 1 and 2, the catheter tubes obtained in Examples 1 to 3 are superior in shape recovery (memory) efficiency and also in flexibility.

## Claims

1. A medical catheter tube, comprising an internal resin layer, a reinforcing material layer formed on the internal resin layer and an external resin layer formed on the reinforcing material layer,
wherein the reinforcing material layer comprises twisted yarns.

2. The medical catheter tube according to Claim 1, wherein single yarns forming the twisted yarns comprise a nonmetal wire.

3. The medical catheter tube according to Claim 2, wherein the nonmetal wire is a glass fiber.

4. The medical catheter tube according to any one of Claims 1 to 3, wherein the shape recovery (memory) rate in the folded region after folding 180° is 90% or more.

5. The medical catheter tube according to any one of Claims 2 to 4, wherein the nonmetal wire contains a radiopaque element.

6. The medical catheter tube according to Claim 5, wherein the radiopaque element is one or more elements selected from barium, tantalum, tungsten, iridium, platinum, gold, and bismuth.

7. The medical catheter tube according to any one of Claims 1 to 6, wherein the single yarns for the twisted yarn comprise a metal wire.

8. The medical catheter tube according to any one of Claims 1 to 7, wherein the single yarns for the twisted yarn comprise a metal wire and a nonmetal wire.

9. The medical catheter tube according to any one of Claims 2, 5, 6 and 8, wherein the nonmetal wire has a tensile strength of 13.2 cN/dtex or more and a structure having a melting liquid crystal polymer as core and a flexible polymer as sheath.

10. The medical catheter tube according to any one of Claims 1 to 3, and 5 to 9, wherein the shape recovery (memory) rate in the folded region after folding 180° is 75% or more.
